# EUROPÄISCHE PATENTSCHRIFT

(11) **EP 2 323 705 B1**
(45) Veröffentlichungstag und Bekanntmachung des Hinweises auf die Patenterteilung: **05.10.2016**
(21) Anmeldenummer: 09744605.8
(22) Anmeldetag: 28.08.2009
(51) Int. Cl.: A61L 27/30, A61L 27/56

(54) **IMPLANTAT UND VERFAHREN ZU SEINER HERSTELLUNG SOWIE DEREN VERWENDUNG**
IMPLANT AND METHOD FOR THE PRODUCTION THEREOF AND USE THEREOF
IMPLANT ET PROCÉDÉ POUR SA PRODUCTION ET SON UTILISATION

(30) Priorität: 05.09.2008 DE 102008046198
(43) Veröffentlichungstag der Anmeldung: 25.05.2011
(73) Patentinhaber: Verein zur Förderung von Innovationen durch Forschung, Entwicklung und Technologietransfer e.V. (Verein INNOVENT e.V.), 07745 Jena (DE)
(72) Erfinder: SCHMIDT, Jürgen, 07745 Jena (DE); SCHNABELRAUCH, Matthias, 07749 Jena (DE); SCHRADER, Christian, 07743 Jena (DE); WEISSER, Jürgen, 99092 Erfurt (DE); HENNING, Angelika, 07751 Zöllnitz (DE); WYRWA, Ralf, 07751 Rothenstein (DE)
(74) Vertreter: Oehmke, Volker
(86) Internationale Anmeldenummer: PCT/DE2009/050047
(87) Internationale Veröffentlichungsnummer: WO 2010/025720

(56) Entgegenhaltungen:
- WO-A1-2006/004297
- US-A- 5 556 645
- US-A1- 2006 018 945

## Beschreibung

Die vorliegende Erfindung betrifft ein Implantat, das wenigstens teilweise in den Knochen eingesetzt wird. Weiterhin betrifft die vorliegende Erfindung ein Verfahren zur Beschichtung eines Implantatgerüsts sowie deren Verwendung.

Verletzte oder beschädigte Knochengewebeteile werden bei Eutheriainsbesondere beim Menschen im günstigsten Fall durch körpereigenes Knochengewebe wiederhergestellt oder mechanisch verstärkt. Dies ist aus verschiedenen Gründen nicht immer möglich. Deshalb wird häufig synthetisches Material als temporäres d. h. bioabbaubares bzw. postoperativ entfernbares oder permanentes Ersatzmaterial verwendet.

Im Knochengewebe verankerte Implantate dienen hauptsächlich dem temporären oder permanenten Ersatz oder dem Support von abnutzungs-, mangelerscheinungs-, unfall- oder krankheitsgeschädigten oder sonst degenerierten Teilen des Skeletts. Als Implantat wird normalerweise ein künstliches, chemisch stabiles Material bezeichnet, welches als plastischer Ersatz oder zur mechanischen Verstärkung in den Körper eingebracht wird [vgl. z. B. Roche Lexikon Medizin, Urban & Fischer, (Hrsg.); 5. Aufl. 2003], wobei die mechanischen Eigenschaften und das Implantatdesign die Hilfs-, Stütz- und Ersatzfunktion im Körper bestimmen. Beispielsweise haben sich Kniegelenk- und Hüftprothesen, WirbelsäulenDimplantate sowie Materialien zur Versorgung von Knochenfrakturen (Osteosynthese) seit vielen Jahren erfolgreich im klinischen Einsatz bewährt. Aufgrund der stabilisierenden, lastaufnehmenden und/oder -übertragenden Funktion solcher Implantate, werden sehr oft Metalle oder metallische Legierungen als geeignete Werkstoffe für deren Herstellung eingesetzt. Typische Implantatmetalle bzw. metallische Legierungen, die im Knochengewebebereich eingesetzt werden, sind Titan und geeignete Legierungen davon, Edelstahl oder auch Kobalt-Chrom-Legierungen. Für eine stabile Implantatverankerung und die Implantatverträglichkeit an der Grenzfläche zwischen Implantatoberfläche und angrenzendem Gewebe kommt der Implantatoberfläche eine besonders große Bedeutung zu. Durch eine Funktionalisierung der Implantatoberfläche können die Implantatverankerung und die Implantatverträglichkeit verbessert sowie auch der Heilungsprozess beschleunigt werden.

Bei der Oberflächenbehandlung und Oberflächenstrukturierung kommen verschiedenste Methoden zur Anwendung, die z. B. in "Titanium in Medicine, Material Science, Surface Science, Engineering, Biological Responses and Medical Applications Series: Engineering Materials" [Eds.: Brunette, D. M., Tengvall, P., Textor, M., Thomsen, P.] und in darin genannten Referenzen beschrieben werden.

Bewährt haben sich im Einsatz besonders calciumphosphathaltige Oberflächenschichten, die durch thermisches Spritzen, Sol-Gel- oder elektrochemische Verfahren auf ImplantatDoberflächen aufgebracht werden können. Calciumphosphat-Beschichtungen zeigen aufgrund ihrer chemischen Ähnlichkeit mit der mineralischen Knochenphase eine sehr gute Biokompatibilität. In Abhängigkeit von der Art der Calciumphosphat-Beschichtung und der damit verbundenen Löslichkeit können Calcium- und Phosphationen im neu gebildeten Knochen integriert werden. Dadurch wird ein stabilerer Verbund zwischen Implantat und umliegendem Knochengewebe ermöglicht. Eine optimierte offen-poröse Struktur der Calciumphosphat-Beschichtung begünstigt zusätzlich das Einwachsen und die Verankerung von Knochenzellen. Diese als Osteokonduktivität bezeichnete Eigenschaft der Beschichtung fördert eine schnelle Stabilisierung des Implantats im umliegenden Gewebe.

Ein wesentlicher Nachteil von Calciumphosphat-Beschichtungen ist darin zu sehen, dass diese nicht osteogen wirken, also den Heilungsprozess des Knochengewebes nicht aktiv unterstützen. Daher wurden in den letzten Jahren vermehrt große Anstrengungen unternommen, Implantatoberflächen mit Substanzen zu beschichten, die eine osteogene und/oder osteoinduktive Wirkung entfalten können. Substanzen mit osteoinduktiver Wirkung sind beispielsweise Wachstumsfaktoren, wie die sogenannten Bone Morphogenetic Proteins (BMPs). Auch niedermolekulare Substanzen sind in der Lage, die Prozesse der Knochenbildung und der Knochenresorption zu beeinflussen. Als solche osteogene Substanzen sind aus der Literatur beispielsweise Bisphosphonate (z. B. Alendronat), Statine, wasserlösliche Strontiumsalze (z. B. Strontiumranelat) oder wasserlösliche Galliumsalze (z. B. Gallium(III)nitrat) bekannt. Gegenwärtig werden diese Substanzen in der Osteoporose-Behandlung teilweise bereits eingesetzt [Biskoping, D. M., Expert Opin. Invest. Drugs 12 (2003), 611-621; Bockman, R. S. et al., J. Bone Miner. Res. 4 (1989), 167]. Solche niedermolekularen Verbindungen sind zur Erzielung osteogener Eigenschaften von Implantatoberflächen sehr interessant, da sie im Vergleich zu Proteinen wie BMP in der Regel weniger empfindlich gegenüber etablierten Sterilisationsverfahren für Implantate sind. Unter den niedermolekularen Substanzen mit potentiell osteogener Wirkung sind Galliumsalze von besonderem Interesse. Bekannt ist, dass Galliumionen knochenbildende Zellen (Osteoblasten) aktivieren und eine anabole Aktivität (knochenwachstumsfördernd) besitzen. Auch inhibiert Gallium die Sekretion von IL-6 sowie anderen Osteoklasten-aktivierenden Zytokinen. Die Knochenresorption durch knochenabbauende Zellen (Osteoklasten) kann durch Galliumionen reduziert werden, was höchstwahrscheinlich auf die Inhibierung vakuolärer ATPasen zurückzuführen ist [L. R. Bernstein: Pharmacological Reviews, 50, 4 (1998), 665-682]. Des Weiteren wurde gefunden, das sich Gallium zur Behandlung tumorbedingter Hyperkalzämie eignet und die damit verbundene Calcium-resorption am Knochen reduziert wird [W. P. Raymond et al., J. Clin. Invest. (1984), 73, 1487-1490]. Wasserlösliche Galliumsalze wie Gallium(III)nitrat und Gallium(III)acetat sind daher als Pharmaka zur systemischen Applikation bei knochentumorbedingter Hyperkalzämie, Morbus Paget und Osteoporose [R. S. Bockuran et al., Semin. Arthritis Rheum. 23 (1994), 268-269] sowie zur topischen Behandlung von Arthritis [G. Eby, Medical Hypotheses (2005) 65, 1136-1141] bereits klinisch getestet. Der 67 Galliummetabolismus wurde aufgrund der Anwendung von Ga in der Radionuclidmedizin bereits eingehend untersucht. Nachteilig wirkt sich bei einer oralen Gabe wasserlöslicher Galliumsalze die geringe Absorption im Intestinaltrakt aus. Es erfolgt nur eine geringe Aufnahme über die Duodenalschleimhaut und überwiegend ein Rücktransport in den Darm [P.O. Ganrot, Environmental Health Perspectives (1986), 65, 363-441]. Ein lokales Depot am Wirkungsort mit langsamer Freisetzung der Galliumionen wäre von großem Vorteil.

Es wurden in der Vergangenheit zahlreiche Versuche unternommen, die Osteointegration verbessernde hoch- oder niedermolekulare Substanzen auf unbeschichteten sowie auf Calciumphosphat- oder andersartig beschichteten Implantatoberflächen zu immobilisieren. Da eine rein adsorptive Immobilisierung auf Implantatoberflächen, beispielsweise durch Tauchen oder Besprühen derselben mit geeigneten Lösungen, der die Osteointegration verbessernden Substanzen den Nachteil einer schnellen Auswaschung der Substanzen aus der Oberfläche mit sich bringt, wurden unterschiedliche Verfahren einer dauerhaften Immobilisierung vorgeschlagen. Beispiele sind die kovalente Fixierung von Bisphosphonaten auf Calciumphosphat-Oberflächen [Peter B. et al., Local delivery of bisphosphonate from coated orthopedic implants increases implants mechanical stability in osteoporotic rats., J Biomed Mater Res A. 2006 Jan;76(1):133-143], die Beschichtung von metallischen Implantaten mit Suspensionen aus resorbierbaren Polymeren und BMP [Schmidmaier et al., Collective review: bioactive implants coated with poly(D,L-lactide) and growth factors IGF-I, TGF-betal, or BMP-2 for stimulation of fracture healing, J Long Term Eff Med Implants. 2006;16(1):61-69]. Wesentliche Nachteile all dieser Verfahren sind jedoch darin zu sehen, dass die Aufbringung der die Osteointegration verbessernden Komponente mit einem hohen technischen Aufwand verbunden ist und zusätzliche Hilfsstoffe wie Polymere, organische Lösungsmittel oder Kupplungsagenzien benötigt werden, die das Einwachsverhalten der Implantate negativ beeinflussen können. Zudem besitzen diese Schichten nur eine geringe mechanische Stabilität. Wie bereits erwähnt bestehen außerdem Probleme bei der thermischen Behandlung oder γ-Bestrahlung zur Sterilisation, was zur Zerstörung der Beschichtung, Inaktivierung von Proteinstrukturen oder zu toxischen Abbauprodukten führen kann. Um diese genannten Probleme zu umgehen, wird in der US 5,556,645 A die Möglichkeit der Imprägnierung mit galliumhaltigen Verbindungen beschrieben. Jedoch können beim Tränken mit einer galliumhaltigen Lösung nur sehr dünne Schichten erhalten werden. Dickere Beschichtungen im µm-Bereich sind mit dem Imprägnieren nicht erreichbar. Zudem lassen sich metallische Implantatoberflächen nur schlecht imprägnieren. Nachteilig beim Imprägnieren ist auch, dass eine Einstellung von Schichtdicken und Porositäten nicht möglich ist. Die erzeugten Schichten sind zudem nicht stabil und werden bei der mechanischen Scherbelastung einer Implantation, die im kPa bis MPa liegen kann, abgetragen, was zu einem undefinierten Gallium-Release führt und therapeutisch relevante Galliumionen-Konzentrationen über längere Zeit nicht erreicht werden können.

Der Erfindung liegt die Aufgabe zugrunde, unter Vermeidung der Nachteile des Standes der Technik eine Möglichkeit zur Herstellung eines Implantats anzugeben, welches sich im eingesetzten Zustand insbesondere durch eine gute, komplikationslose Osteointegration auszeichnet.

Erfindungsgemäß wird die Aufgabe dadurch gelöst, dass mindestens ein Teil der Oberfläche einer Implantatstruktur mit einer festhaftenden, porösen Beschichtung versehen wird, die als wirksamen Schichtbestandteil Galliumionen in Form eines variablen Anteils einer in wässrigen und/oder physiologischen Medien schwerlöslichen Galliumverbindung in Form von Galliumphosphat enthält. Das Implantat besteht aus einem Implantatgerüst und einer mindestens teilweise darauf befindlichen Beschichtung, dadurch gekennzeichnet, dass die Beschichtung auf dem Implantatgerüst, dessen Oberfläche glatt, strukturiert und/oder porös ausgebildet ist, Galliumionen in Form von in wässrigen und/oder physiologischen Medien schwerlöslichen Galliumverbindungen enthält, wobei der Galliumgehalt in der Beschichtung 1 bis 50 Atomprozente beträgt und die Galliumverbindungen in Form von Galliumphosphat vorliegen, wobei das Galliumphosphat in amorpher oder nanokristalliner Form vorliegt, eine Dicke von 4 7 0,1 bis 50 µm aufweist, und eine poröse Struktur mit einer Porendichte von 10 bis 10 Poren/ mm² und einer durchschnittliche Porengröße zwischen 0,1 und 10 µm aufweist.

Ein wesentlicher Vorteil der Erfindung ist es, dass bei der anodischen Oxidation schon bei geringen Galliumionenkonzentrationen unter speziellen Bedingungen eine Anreicherung von Gallium in Form von Galliumphosphat in der Beschichtung stattfindet und auch reine Galliumphosphatschichten generierbar sind. Die neuartigen galliumhaltigen Beschichtungen zeichnen sich durch Poren aus, die in einer Größenordnung von 0.1 bis 10 µm liegen, und die damit für eine Osteointegration besonders geeignet sind.

Diese Beschichtungen zeichnen sich ferner dadurch aus, dass sie unabhängig vom Galliumgehalt von tierischen Zellen ohne Inhibition des Zellwachstums besiedelt werden. Es kann sich dabei auch um eine reine GaPO₄-Schich handeln.

Die durch die Erfindung erreichten Vorteile sind im Wesentlichen darin zu sehen, dass Dank der erfindungsgemäßen porenhaltigen Beschichtung der Implantatstruktur der Knochen schneller einwachsen kann. Dabei erfolgt durch die in der Beschichtung in Form von in Wasser schwerlöslichen Galliumverbindungen vorhandenen Galliumionen über lange Zeit eine Stimulierung knochenbildender Zellen (Osteoblasten), so dass eine verbesserte Integration des Implantats in das umliegende Gewebe gegeben ist. Da die Galliumionen in Form einer schwerlöslichen Galliumverbindung in die Beschichtung integriert sind, ist die Konzentration an gelösten, frei beweglichen Galliumionen in der Umgebung der Implantatgrenzfläche gering, so dass zwar ausreichend Galliumionen zur Stimulierung des Knochenwachstums vorhanden sind, aber keine Gefahr einer Überdosierung und damit einer unerwünschten Nebenwirkung von Galliumionen auf das umgebende Gewebe besteht. Eine gewebeschädigende Konzentration oberhalb 50 µM wird praktisch nicht erreicht.

Die poröse Beschichtung kann ebenfalls dazu dienen, in bekannter Weise Wirkstoffe bzw. Wirkstoffkombinationen - gegebenenfalls in einer Matrix eingebettet - aufzunehmen, wodurch zusätzlich z. B. wachstumsfördernde, entzündungshemmende oder osteointegrationsfördernde Substanzen freigesetzt werden, sodass diese zusätzlichen Wirksubstanzen die entsprechenden Wirkungen unterstützend lokal entfalten.

Der Galliumanteil in der Beschichtung beträgt 1 bis 50 Atomprozente, vorzugsweise 5 bis 30 Atomprozente.

In einer weiteren Ausführungsform der Erfindung enthält die Beschichtung Galliumionen in Form von amorphem oder nanokristallinem Galliumphosphat, welches zusätzlich die Funktion von Kristallisationskeimen bei der Knochenneubildung besitzt.

Weiterhin ist es von Vorteil, wenn die galliumhaltige Beschichtung amorphe und/ oder kristalline Calciumphosphat-Phasen enthält.

In einer weiteren Ausführungsform der Erfindung enthält die galliumhaltige Beschichtung einen Anteil von 20 bis 95 Atomprozenten Metalloxid bestehend aus einem oder mehreren Metallen oder Mischungen unterschiedlicher Metalloxide.

Als besonders geeignetes Metalloxid hat sich Titandioxid erwiesen.

Die Dicke der Beschichtung ist zwischen 0,5 bis 50 µm stark, vorzugsweise maximal 10 µm. Eine derartige, dünne Beschichtung verfügt über gute Hafteigenschaften auf dem Substrat und ist relativ stabil gegenüber mechanischer, insbesondere Scherbeanspruchung.

Von besonderem Vorteil ist die Porosität der Beschichtung, wobei die Porendichte zwischen 10⁴ und 10⁷ Poren/mm² und die durchschnittliche Größe der Poren zwischen 0,2 und 10 µm liegt.

Die erfindungsgemäße Beschichtung wird vorzugsweise auf Implantatgerüste aufgebracht, die aus in der Medizin- und Dentaltechnik üblichen Metallen oder Metalllegierungen bestehen, vorzugsweise solchen Metallen oder Metalllegierungen, die eines oder mehrere der Elemente Ti, Ta, Nb, Zr, Al, Mg oder deren Legierungen enthalten. Die Implantatgerüste können dabei eine beliebige Form und Oberflächenmorphologie besitzen und die Beschichtung kann das Implantatgerüst vollständig oder teilweise umfassen.

Die erfindungsgemäße Beschichtung wird mittels anodischer Oxidationsverfahren, vorzugsweise dem Verfahren der plasmachemischen Oxidation auf die zu beschichtende Implantatstruktur aufgebracht. Der in diesem Verfahren eingesetzte wässrige Elektrolyt enthält dabei Gallium-, Phosphat- und gegebenenfalls weitere Ionen, wie z. B. Calcium, in Form geeigneter wasserlöslicher Salze. Der für die plasmachemische Oxidation verwendete Elektrolyt kann darüber hinaus weitere, den Prozess der Beschichtung verbessernde Zusätze wie Komplex- oder Chelatbildner oder den pH-Wert und/oder die Elektrolytleitfähigkeit einstellende Zusätze enthalten. Die Beschichtung entsteht durch Reaktion zwischen der metallischen Implantatstruktur und den Komponenten des Elektrolyten unterstützt durch plasmachemische Reaktionen.

Die Erfindung soll nachstehend anhand von Ausführungsbeispielen näher erläutert werden.

### Beispiel 1

### Galliumphosphat-Schicht

Es wird ein wässriger Elektrolyt mit 50 g/l (0,13 mol/l) Ethylendiamintetraessigsäure-di-Natrium-monohydrat (Na₂-EDTA x H₂O), 20 g/l (0,174 mol/l) AmmoniumdiDhydroDgenphosphat (NH₄H₂PO₄) sowie 4 g/l (0,02 mol/l) Galliumnitratmonohydrat (Ga₂(NO₃)₃x H₂O) bereitgestellt. Weiterhin werden 20 ml/l (0,29 mol/l) Phosphorsäure (H₃PO₄) sowie 50 ml/l (0,73 mol/l) Ethylendiamin (C₂H₈N₂) zugesetzt. In diesem Elektrolyt wird anschließend eine Edelstahlkathode eingeführt und elektrisch mit einer äußeren Stromversorgungsquelle kontaktiert. Die zu beschichtende Implantatstruktur aus TiAl6V4 (10x10x1 mm³) wird anodisch kontaktiert und mit der Stromversorgung verbunden. Nach dem Einschalten der Stromversorgung wird bei einer konstanten Stromdichte von 1 A/dm² die Badspannung (Startpunkt = 0 Volt) erhöht. Die Elektrolyttemperatur beträgt zwischen 10°C und 40 °C. Es kommt gepulster Gleichstrom mit einer Frequenz von 1 kHz bei einem Tastverhältnis von 1:1 zur Anwendung. Bei Badspannungen von ca. 165 V zeigen sich erste plasmachemische Oxidationseffekte, die anhand von visuell sichtbaren Funkenentladungen an der Anodenoberfläche verfolgt werden können. Wenn die Badendspannung einen Wert von ca. 210 V erreicht hat, lässt man die Spannung nicht weiter steigen. Infolge dessen kommt es zu einem Absinken des Stromes. Wenn dieser die Hälfte des Ausgangswertes erreicht hat, wird der Beschichtungsprozess durch Ausschalten der Stromversorgung beendet. Das Implantat wird aus dem Elektrolyt entfernt, mehrmals in destilliertem Wasser gespült und an der Luft getrocknet. Die Implantatoberfläche weist eine charakteristische mikroporöse Oberfläche bei Schichtdicken zwischen 4 µm bis 5 µm auf. Die energiedispersive Röntgenanalyse der Schicht zeigt deutliche Gehalte an Ga.

### Beispiel 2

### Gallium/Calcium-Phosphat-Schicht

Es wird ein wässriger Elektrolyt mit 50 g/l (0,12 mol/l) Ethylendiamintetraessigsäure-Calcium-di-Natrium-dihydrat (Ca-Na₂-EDTA x 2.H₂O), 20 g/l (0,174 mol/l) AmmoniumDdihydrogenphosphat (NH₄H₂PO₄) sowie 4 g/l (0,02 mol/l) Galliumnitratmonohydrat (Ga₂(NO₃)₃ x H₂O) bereitgestellt. Weiterhin werden 20 ml/l (0,29 mol/l) Phosphorsäure (H₃PO₄) sowie 50 ml/l (0,73 mol/l) Ethylendiamin (C₂H₈N₂) zugesetzt. In diesem Elektrolyten wird anschließend eine Edelstahlkathode eingeführt und elektrisch mit einer äußeren Stromversorgungsquelle kontaktiert. Die zu beschichtende Implantatstruktur aus TiAl6V4 (10x10x1 mm³) wird anodisch kontaktiert und mit der Stromversorgung verbunden. Nach dem Einschalten der Stromversorgung wird bei einer konstanten Stromdichte von 1 A/dm² (Startpunkt = 0 Volt) erhöht. Die Elektrolyttemperatur beträgt zwischen 10°C und 40 °C. Es kommt gepulster Gleichstrom mit einer Frequenz von 1 kHz bei einem Tastverhältnis von 1:1 zur Anwendung. Bei Badspannungen von ca. 130 V zeigen sich erste plasmachemische Oxidationseffekte, die anhand von visuell sichtbaren Funkenentladungen an der Anodenoberfläche verfolgt werden können. Wenn die Badendspannung einen Wert von ca. 165 V erreicht hat, lässt man die Spannung nicht weiter steigen. Infolge dessen kommt es zu einem Absinken des Stromes. Wenn dieser die Hälfte des Ausgangswertes erreicht hat, wird der Beschichtungsprozess durch Ausschalten der Stromversorgung beendet. Das Implantat wird aus dem Elektrolyt entfernt, mehrmals in destilliertem Wasser gespült und an der Luft getrocknet. Die Implantatoberfläche weist eine charakteristische mikroporöse Oberfläche bei Schichtdicken zwischen 1 µm bis 2 µm auf. Die energiedispersive Röntgenanalyse der Schicht zeigt deutliche Gehalte an Ca, Ga und P.

### Beispiel 3

### Galliumphosphat-Schicht

Es wird ein wässriger Elektrolyt mit 10 g/l (0,04 mol/l) Galliumnitrat (Ga(NO₃)₃. x·H₂O), 50 g/l (0,4 mol/l) Oxalsäure-dihydrat (H₂C₂O₄ · 2H₂O) sowie 24 g/l (0,2 mol/l) NatriumdiDhydrogenphosphat (NaH₂PO₄) bereitgestellt. In diesem Elektrolyten wird anschließend eine Edelstahlkathode eingeführt und elektrisch mit einer äußeren Stromversorgungsquelle kontaktiert. Die zu beschichtende Implantatstruktur aus TiAl6V4 bzw. Reintitan (40x40x1 mm³) wird anodisch kontaktiert und mit der Stromversorgung verbunden. Nach dem Einschalten der Stromversorgung wird bei einer konstanten Stromdichte von 1 A/dm² die Badspannung (Startpunkt = 0 Volt) erhöht. Die Elektrolyttemperatur beträgt zwischen 10°C und 40 °C. Es kommt gepulster Gleichstrom mit einer Frequenz von 1 kHz bei einem Tastverhältnis von 1:1 zur Anwendung. Bei Badspannungen von ca. 180 V zeigen sich erste plasmachemische Oxidationseffekte, die anhand von visuell sichtbaren Funkenentladungen an der Anodenoberfläche verfolgt werden können. Wenn die Badendspannung einen Wert von ca. 280 V erreicht hat, lässt man die Spannung nicht weiter steigen. Infolge dessen kommt es zu einem Absinken des Stromes. Wenn dieser die Hälfte des Ausgangswertes erreicht hat, wird der Beschichtungsprozess durch Ausschalten der Stromversorgung beendet. Das Implantat wird aus dem Elektrolyt entfernt, mehrmals in destilliertem Wasser gespült und an der Luft getrocknet. Die Implantatoberfläche weist eine charakteristische mikroporöse Oberfläche bei Schichtdicken zwischen 1 µm bis 2 µm auf. Die energiedispersive Röntgenanalyse der Schicht zeigt deutliche Gehalte an Ga und P.

Nach der plasmachemischen Oxidation wurden die Beschichtungen der Implantate mittels Röntgenfluoreszenzanalyse (RFA) untersucht. Die Ergebnisse der RFA zeigen für die Galliumphosphat-(GaP)-Beschichtung einen deutlichen Galliumpeak. Bei der plasmachemischen Oxidation in einem Elektrolyten, der Gallium- und Calciumionen enthält, können Schichten erzeugt werden, in der beide Elemente vorhanden sind. Die RFA zeigt in einer Gallium-Calcium-Phosphat (Ga-CaP)-Beschichtung deutliche Signale für Ca und Ga.

Die Bestimmung der Schichtdicke an Querschliffen unverdichteter galliumphosphathaltiger Beschichtungen auf den Implantaten (TiAl6V4, 10x10x1 mm³) mittels plasmachemischer Oxidation ergab Werte von 3 bis 50 µm.

Die Porosität der mittels plasmachemischer Oxidation beschichteten Implantate wurden mit Hilfe der Rasterelektronenmikroskopie (REM) untersucht. Die REM-Aufnahmen der Oberfläche der Beschichtungen weisen je nach Herstellungsprozess Poren in Größen kleiner 1 µm bis maximal 10 µm auf.

Ein nach Beispiel 3 GaPO₄-beschichteter Probekörper aus Reintitan wurde 10 Tage bei 37°C mit 1,5 ml Reinstwasser überschichtet. Anschließend wurden 100 µl der Lösung abgenommen und mit bekannten Analytikmethoden qualitativ auf herausgelöstes Gallium untersucht. Dabei konnten Galliumionen nachgewiesen werden, wobei u. a. der Nachweis mittels Alizarin S durchgeführt wurde. Die Blindprobe verlief negativ.

Um eine Eluation von Ti-Ionen auszuschließen, wurde das Implantat mittels Röntgenfluoreszenzanalyse (RFA) analysiert. Es konnte dabei kein charakteristisches Fluoreszenzsignal von Titan bei 4,5 keV nachgewiesen werden.

Die quantitative Bestimmung ergab eine Menge an freigesetzten Galliumionen, die einer lokalen therapeutischen Dosis entspricht.

Zur Untersuchung der Bioverträglichkeit und der Zytotoxizität wurden AP-Aktivität und Protein-Gesamtmenge kultivierter Zellen auf den Beschichtungen untersucht. Es zeigte sich, dass nach 20 Kultivierungstagen die galliumhaltigen Schichten sowohl hinsichtlich der Gesamtentwicklung der AP-Aktivität als auch hinsichtlich der Relation der AP-Aktivität zur Protein-Gesamtmenge mit der unbeschichteten Kontrollprobe und mit der galliumfreien Calciumphosphatbeschichtung vergleichbar waren. Das ist ein positives Ergebnis, da insbesondere die auf einer nur galliumionenhaltigen Schicht wachsenden Zellen im Mikromilieu theoretisch durchaus lokal hohen und damit zytotoxischen Galliumkonzentrationen ausgesetzt sein könnten. Dass dies nicht der Fall ist, zeigen auch die Ergebnisse der Vitalitätsfärbung. Die Anfärbung der Kulturen nach 21 Tagen auf AP-positive Zellen bestätigte, dass auf den galliumhaltigen Schichten AP-positive Zellen und Zellklone ähnlich der unbeschichteten Kontrollprobe vorlagen.

Durch die plasmachemische Oxidation können poröse, fest haftende bioverträgliche und bioaktive Beschichtungen aus Galliumphosphat und/oder Mischschichten mit anderen Metallen wie z. B. Gallium/Calcium-Phosphat auf metallischen Implantatmaterialien erzeugt werden. Mit Hilfe des Verfahrens werden Beschichtungen zur vollständigen oder teilweisen Ausrüstung von metallischen Oberflächen von Implantaten für den Einsatz im medizinischen sowie im Dentalbereich bereitgestellt, wobei die Oberflächen bevorzugt glatt, strukturiert und/oder porös sind. Die erzeugte Porosität der Beschichtung fördert die Osteointegration. Die Poren können ebenfalls für das Einbringen zusätzlich bioaktiver Substanzen genutzt werden, wodurch eine lokale Freisetzung der Wirkstoffe stattfindet.

Aufgrund des Galliumgehaltes in der Beschichtung besteht trotz des geringen Löslichkeitsproduktes eine langsame Freisetzung von Galliumionen. Diese können in bekannter Weise knochenbildende Zellen (Osteoblasten) aktivieren und eine anabole Aktivität (knochenwachstumsfördernd) entfalten. Ebenso werden durch die Gainduzierte Inhibierung der Sekretion von IL-6 sowie anderen Zytokinen eine Osteoklasten-Aktivierung gehemmt. Die Knochenresorption durch knochenabbauende Zellen (Osteoklasten) kann ebenfalls durch die freigesetzten Galliumionen reduziert werden, was höchstwahrscheinlich auf die Inhibierung vakuolärer ATPasen zurückzuführen ist. Das freigesetzte Gallium eignet sich ebenso in bekannter Weise zur Behandlung tumorbedingter Hyperkalzämie und die damit verbundene Calciumresorption am Knochen. Aufgrund der osteointegrativen, entzündungshemmenden Eigenschaften eignen sich Implantate mit einer solchen Ga-haltigen Beschichtung besonders auch in den Bereichen der Implantationsmedizin, wo der Einsatz herkömmlicher Implantate contraindiziert ist. Daher ist eine Verwendung dieser Implantate im medizinischen und Dentalbereich z. B. bei Osteoporose, Osteodystrophia deformans (Paget-Syndrom), Knochenkrebserkrankungen, bei arthritischen und parodontalen Erkrankungen erfolgtversprechend.

## Patentansprüche

1. Implantat bestehend aus einem Implantatgerüst und einer mindestens teilweise darauf befindlichen Beschichtung, **dadurch gekennzeichnet, dass** die Beschichtung auf dem Implantatgerüst, dessen Oberfläche glatt, strukturiert und/oder porös ausgebildet ist,
- Galliumionen in Form von in wässrigen und/oder physiologischen Medien schwerlöslichen Galliumverbindungen enthält, wobei der Galliumgehalt in der Beschichtung 1 bis 50 Atomprozente beträgt und die Galliumverbindungen in Form von Galliumphosphat vorliegen, wobei das Galliumphosphat in amorpher oder nanokristalliner Form vorliegt,
- eine Dicke von 0,1 bis 50 µm aufweist, und
- eine poröse Struktur mit einer Porendichte von 10⁴ bis 10⁷ Poren/mm² und einer durchschnittliche Porengröße zwischen 0,1 und 10 µm aufweist.

2. Implantat nach Anspruch 1, **dadurch gekennzeichnet, dass** die Beschichtung einen Anteil von 20 bis 95 Atomprozenten Titandioxid enthält.

3. Implantat nach einem der vorhergehenden Ansprüche, **dadurch gekennzeichnet, dass** das Implantatgerüst eines der Elemente Ti, Ta, Nb, Zr, Al, Mg oder deren Legierungen enthält.

4. Implantat nach einem der vorhergehenden Ansprüche, **dadurch gekennzeichnet, dass** in die Poren der Beschichtung ein Wirkstoff oder eine Wirkstoffkombination bzw. diese eingebettet in einer Matrix eingebracht ist.

5. Verfahren zur Herstellung eines Implantats nach einem der Anspüche 1 bis 3, **dadurch gekennzeichnet, dass** das Implantat mindestens teilweise in einem wässrigen, Galliumionen enthaltenden Elektrolyten durch eine anodische Oxidationsreaktion beschichtet wird.

6. Verfahren nach Anspruch 5, **dadurch gekennzeichnet, dass** die anodische Oxidationsreaktion als plasmachemische Oxidation durchgeführt wird.

7. Verfahren nach Anspruch 6, bei dem
- ein wässriger Elektrolyt enthaltend:
- 0,1 bis 0,25 mol/l Ethylendiamintetraessigsäure-di-Natrium-monohydrat (Na₂-EDTA x H₂O),
- 0,05 bis 0,28 mol/l) Ammoniumdihydrogenphosphat (NH₄H₂PO₄),
- 0,01 bis 0,15 mol/l Galliumnitrat-monohydrat (Ga₂(NO₃)₃ x H₂O),
- 0,1 bis 0,35 mol/l Phosphorsäure (H₃PO₄) und
- 0,5 bis 2,4 mol/l Ethylendiamin (C₂H₈N₂)
bereitgestellt wird,
- anschließend eine Edelstahlkathode eingeführt und elektrisch mit einer äußeren Stromversorgungsquelle kontaktiert wird,
- die zu beschichtende Implantatstruktur anodisch kontaktiert und mit der Stromversorgung verbunden wird,
- nach dem Einschalten der Stromversorgung, die einen gepulsten Gleichstrom mit einer Frequenz von 10 bis 2000 Hz bei einem Tastverhältnis von 1:1 liefert, bei einer konstanten Stromdichte von 0,5 bis 5 A/dm² die Badspannung (Startpunkt = 0 Volt) erhöht wird, wobei die Elektrolyttemperatur zwischen 10°C und 40 °C gehalten wird,
- bei erreichen der Badspannung von minimal 150 V bis maximal 350 V wird die Spannung nicht weiter erhöht, infolge dessen es zu einem Absinken des Stromes kommt,
- bei erreichen der Hälfte des Ausgangswertes des Stromes wird der Beschichtungsprozess durch Ausschalten der Stromversorgung beendet und das fertig beschichtete Implantat wird aus dem Elektrolyt entfernt.

## Claims

1. Implant composed of an implant structure and a coating that is located thereon at least partially, **characterized in that** the coating on the implant structure, the surface of which is formed in a smooth, structured and/or porous manner,
- contains gallium ions in the form of gallium compounds that are sparingly soluble in aqueous and/or physiological media, wherein the gallium content in the coating amounts to 1 to 50 atom percent and the gallium compounds are present in the form of gallium phosphate, wherein the gallium phosphate is present in amorphous or nanocrystalline form,
- has a thickness of 0.1 to 50 µm, and
- has a porous structure with a pore density of 10⁴ to 10⁷ pores/mm² and an average pore size between 0.1 and 10 µm.

2. Implant according to claim 1, **characterized in that** the coating contains a proportion of 20 to 95 atom percent of titanium oxide.

3. Implant according to one of the preceding claims, **characterized in that** the implant structure contains one of the elements Ti, Ta, Nb, Zr, Al, Mg or their alloys.

4. Implant according to one of the preceding claims, **characterized in that** an active substance or a combination of active substances or the aforementioned embedded in a matrix are introduced into the pores of the coating.

5. Method for producing an implant according to one of claims 1 to 3, **characterized in that** the implant is coated by an anodic oxidation reaction at least partially in an aqueous electrolyte containing gallium ions.

6. Method according to claim 5, **characterized in that** the anodic oxidation reaction is realized as a plasma-chemical oxidation.

7. Method according to claim 6, in which
- an aqueous electrolyte containing:
- 0.1 to 0.25 mol/l ethylenediaminetetraacetic acid disodium monohydrate (Na₂-EDTA x H₂O),
- 0.05 to 0.28 mol/l ammonium dihydrogen phosphate (NH₄H₂PO₄),
- 0.01 to 0.15 mol/l gallium nitrate monohydrate (Ga₂(NO₃)₃ x H₂O),
- 0.1 to 0.35 mol/l phosphoric acid (H₃PO₄) and
- 0.5 to 2.4 mol/l ethylene diamine (C₂H₈N₂)
is provided,
- afterwards a stainless steel cathode is introduced and electrically contacted with an external power supply source,
- the implant structure to be coated is contacted anodically and linked with the power supply,
- after the power supply, which provides a pulsed direct current with a frequency of 10 to 2000 Hz at a duty cycle of 1:1, has been switched on, the bath voltage (starting point = 0 volt) is increased at a constant current density of 0.5 to 5 A/dm², wherein the electrolyte temperature is held between 10°C and 40°C,
- when the bath voltage of, at a minimum, 150 V to, at a maximum, 350 V is reached, the voltage is not increased any further, resulting in a drop of the current,
- when half of the starting value of the current is reached, the coating process is terminated by switching off the power supply, and the finished coated implant is removed from the electrolyte.

## Revendications

1. Implant composé d'une structure d'implant et d'un revêtement se trouvant là-dessus au moins partiellement, **caractérisé en ce que** le revêtement sur la structure d'implant, dont la surface est formée de manière lisse, structurée et/ou poreuse,
- contient des ions gallium sous la forme de composés de gallium qui sont difficilement soluble dans des milieux aqueux et/ou physiologiques, le teneur en gallium dans le revêtement se montant à 1 à 50 pourcent d'atomes et les composés de gallium étant présents sous la forme de phosphate de gallium, le phosphate de gallium étant présent sous forme amorphe ou nanocristalline,
- a une épaisseur de 0,1 à 50 µm, et
- a une structure poreuse avec une densité de pore de 10⁴ à 10⁷ pores/mm² et une taille de pore moyenne entre 0,1 et 10 µm.

2. Implant selon la revendication 1, **caractérisé en ce que** le revêtement contient une proportion de 20 à 95 pourcent d'atomes d'oxyde de titane.

3. Implant selon une des revendications précédentes, **caractérisé en ce que** la structure d'implant contient un des éléments Ti, Ta, Nb, Zr, Al, Mg ou leurs alliages.

4. Implant selon une des revendications précédentes, **caractérisé en ce qu'**une substance active ou une combinaison de substances actives ou celles-ci encastrées dans une matrice sont introduites dans les pores du revêtement.

5. Procédé pour la production d'un implant selon une des revendications 1 à 3, **caractérisé en ce que** l'implant est revêtu par une réaction d'oxydation anodique au moins partiellement dans un électrolyte aqueux contenant des ions gallium.

6. Procédé selon la revendication 5, **caractérisé en ce que** la réaction d'oxydation anodique est effectuée comme oxydation plasma-chimique.

7. Procédé selon la revendication 6, dans lequel
- un électrolyte aqueux contenant:
- 0,1 à 0,25 mol/l de monohydrate d'acide éthylène diamine tétraacétique-disodium (Na₂-EDTA x H₂O),
- 0,05 à 0,28 mol/l de dihydrogénophosphate d'ammonium (NH₄H₂PO₄),
- 0,01 à 0,15 mol/l de monohydrate de nitrate de gallium (Ga₂(NO₃)₃ x H₂O),
- 0,1 à 0,35 mol/l d'acide phosphorique (H₃PO₄) et
- 0,5 à 2,4 mol/l d'éthylène diamine (C₂H₈N₂)
est prévu,
- puis une cathode d'acier inoxydable est introduite et mise en contact électrique avec une source d'alimentation électrique externe,
- la structure d'implant à revêtir est contactée anodiquement et liée à l'alimentation électrique,
- après l'alimentation électrique, qui fournie un courant continu pulsé avec une fréquence de 10 à 2000 Hz à un rapport cyclique de 1:1, a été mise en marche, la tension de bain (point de départ = 0 volt) est augmentée à une densité de courant constante de 0,5 à 5 A/dm², la température d'électrolyte étant maintenue entre 10 °C et 40 °C,
- quand la tension de bain de 150 V, au minimum, à 350 V, au maximum, est atteinte, la tension n'est plus augmentée, et par conséquent, le courant est réduit,
- quand la moitié de la valeur de départ du courant est atteinte, le processus de revêtement est terminé en coupant l'alimentation électrique, et l'implant revêtu fini est retiré de l'électrolyte.
